# EUROPEAN PATENT SPECIFICATION

(11) **EP 4 216 966 B1**
(45) Date of publication and mention of the grant of the patent: **13.11.2024**
(21) Application number: 21815670.1
(22) Date of filing: 11.11.2021
(51) Int. Cl.: A61K 31/785, A61K 47/38, A61K 47/32, A61K 47/26, A61K 47/12, A61K 47/10, A61K 47/36, A61K 47/02, A61K 47/18, G02C 7/04, A61P 31/04, A61P 31/02, A61P 27/02, G02C 7/00, A61K 9/00, A61K 9/06, A61K 9/08

(54) **FORMULATION BASED ON POLYHEXAMETHYLENE BIGUANIDE FOR USE IN THE TREATMENT OF ACANTHAMOEBA KERATITIS AND/OR FUNGAL INFECTIONS**
FORMULIERUNG BASIEREND AUF POLYHEXAMETHYLENBIGUANIDE ZUR VERWENDUNG IN DER BEHANDLUNG VON ACANTHAMOEBA KERATITIS UND/ODER PILZINFEKTIONEN
FORMULATION DU BIGUANIDE DE POLYHÉXAMETHYLÈNE DESTINÉE AU TRAITEMENT DE LA KÉRATITE D'ACANTHAMOEBE ET/OU DES MYCOSES

(30) Priority: 12.11.2020 IT 202000027155
(43) Date of publication of application: 02.08.2023
(73) Proprietor: SIFI S.p.A., 95025 Aci Sant'Antonio (CT) (IT)
(72) Inventor: MOSCHETTI, Valeria, 95029 Viagrande (CT) (IT); PAPA, Vincenzo, 95027 San Gregorio Di Catania (CT) (IT); SUDANO ROCCARO, Andrea, 95024 Acireale (CT) (IT); SPINA, Donato, 95123 Catania (IT); ABBATE, Ilenia, 95024 Acireale (CT) (IT)
(74) Representative: Calogero, Ida
(86) International application number: PCT/IB2021/060451
(87) International publication number: WO 2022/101821

(56) References cited:
- EP-A1- 0 788 797
- US-A1- 2007 140 897
- SCHUSTER F: "Opportunistic amoebae: challenges in prophylaxis and treatment", DRUG RESISTANCE UPDATES, vol. 7, no. 1, 1 February 2004 (2004-02-01), GB, pages 41 - 51, XP055899712, ISSN: 1368-7646, DOI: 10.1016/j.drup.2004.01.002
- VINCENZO PAPA: "Acanthamoeba keratitis therapy: time to cure and visual outcome analysis for different antiamoebic therapies in 227 cases", BR J OPHTHALMOL, vol. 104, no. 4, 10 August 2019 (2019-08-10), pages 575 - 581, XP093168297, Retrieved from the Internet <URL:https://pubmed.ncbi.nlm.nih.gov/31401556/>
- PAPA, VINCENZO ET AL.: "Ocular safety of high doses polyhexanide (PHMB) in healty volunteers", INVESTIGATIVE OPHTHALMOLOGY & VISUAL SCIENCE, vol. 58, 5170, 1 June 2017 (2017-06-01), XP009528421
- BOUATTOUR YASSINE ET AL: "Stability of an ophthalmic formulation of polyhexamethylene biguanide in gamma-sterilized and ethylene oxide sterilized low density polyethylene multidose eyedroppers", vol. 6, 18 April 2018 (2018-04-18), pages e4549, XP055820215, Retrieved from the Internet <URL:https://peerj.com/articles/4549.html> DOI: 10.7717/peerj.4549

## Description

The present invention relates to a stable formulation based on polyhexamethylene biguanide (PHMB) suitable for administration at an ophthalmic level, a process for its preparation and relative dosage regimen effective for the treatment of *Acanthamoeba* keratitis, and in particular for the eradication of cysts.

*Acanthamoeba* keratitis is an infection of the cornea, the transparent tissue that lines the front part of the eye. The infection is caused by *Acanthamoeba*, a microorganism generally present both in streams and water basins (lakes, oceans and rivers), and in home tap water, swimming pools, hydromassage tubs, in the soil and in the air.

*Acanthamoeba* keratitis is more common in contact-lens wearers [1], but anyone with a corneal lesion can become susceptible to developing this serious eye infection.

In general, *Acanthamoeba* has a life cycle in two phases: an active form (when the microorganism feeds and replicates) and a dormant form (when the microorganism protects itself from possible attacks by turning into a cyst). Cysts are the actual target for the development of effective antiamebic drugs.

Co-infection with bacterial species is common in patients with *Acanthamoeba* keratitis. Specifically, *Staphylococcus* spp and *Pseudomonas aeruginosa* are the most common co-isolated bacterial pathogens [2,3]. In particular, for the latter microorganism an *in vivo* study suggests that the presence of bacteria is an essential factor for the development and severity of *Acanthamoeba* keratitis [4].

*Acanthamoeba* keratitis can be extremely painful as the cornea is one of the most sensitive organs in the human body, with the highest density of pain receptors and the products used for treating *Acanthamoeba* keratitis can be quite difficult to bear and, in some cases, they can also irritate the surface of the cornea. About 25% of cases of *Acanthamoeba* keratitis treated even end up with a corneal transplantation. Without treatment, the disease progresses to blindness as a result of corneal vascularization and corneal scarring or perforation.

To date, there are no drugs approved for the treatment of *Acanthamoeba* keratitis. Biguanides and diamidines have shown efficacy for *Acanthamoeba* keratitis in a number of clinical cases, although the treatment regimens and concentration of these drugs have been developed empirically.

Among the different options available, treatment with polyhexamethylene biguanide (PHMB) in an ophthalmic formulation at a concentration of 0.02% alone [5] or in combination with chlorhexidine 0.02%, propamidine 0.1% or desomidine 0.1% is the most promising choice in the treatment of patients suffering from *Acanthamoeba* keratitis.

Current therapies for *Acanthamoeba* keratitis are lengthy and challenging. They start with hourly doses during the day, avoiding the night, for about 1-2 weeks, then reducing according to the response. Although every patient is different, those who have had an early diagnosis and have started receiving adequate treatment immediately, can generally expect from 3 to 6 months of therapeutic treatment.

Furthermore, the repeated and prolonged use of antibiotics and disinfectants, such as those mentioned above, risks significantly altering the composition of the conjunctival flora by increasing the percentage of pathogens such as *Staphylococcus epidermidis* to the detriment of the normal commensal flora and this can have significant clinical implications as *Staphylococcus epidermidis* is one of the main causes of eye diseases such as conjunctivitis, keratitis and endophthalmitis [6].

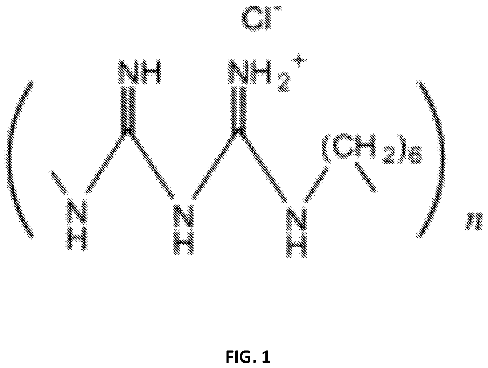

PHMB is composed of repeating (n) times biguanide units connected by hexamethylene chains, which create a cationic and amphipathic structure (Figure 1).

PHMB-based preparations are mixtures of biguanide-based polymers with a molecular weight that can vary from 400 to 8,000 amu and a variable polymerization degree wherein n can range from 2 to 40.

The action mechanism of PHMB has been the subject of various studies in *in vitro* experimental models. The SUV membrane is a system that mimics the chemistry of the cell membrane of the human corneal epithelium. When PHMB interacts with the double layer of the SUV membrane, the cooperation between the phospholipids increases, the water is excluded from the double layer of acyl chains and polar heads, the region with the acyl chains becomes less dynamic and selective, and the PHMB is absorbed by the surface of the double layer [7]. The cationic nature of PHMB allows interaction with anionic polymers, such as DNA. In literature, it has been indicated that PHMB interacts with DNA in aqueous solution forming a complex between the two species. The reaction involves the electrostatic interaction between the cationic biguanide groups and the anionic phosphate groups of the DNA deoxyribonucleotides in a molar ratio of approximately 1: 1 [8].

The structure of *Acanthamoeba*, characterized by the exposure of binding sites to the DNA, allows an easier access of the PHMB to the DNA of the protozoan, facilitating the chromatin condensation process and the destruction of the same microorganism, as already documented for other bacteria such as *Escherichia Coli, Salmonella enteritidis* and *Staphylococcus aureus* [9]. Although these studies are not specific for *Acanthamoeba,* they have been useful to inventors in identifying the optimal conditions in which PHMB binds to the DNA of the protozoan and exerts its best e Phase efficacy in the treatment of infectious keratitis.

In a previous I clinical study of Papa V. et al. [10], it has been shown that even higher PHMB concentrations of 0.04%, 0.06%, 0.08% are safe and well-tolerated in healthy volunteers [8]. The pH and osmolarity of the formulation are not described, much less the molecular weight and the polydispersion index of the PHMB polymer used. The concentration of PHMB alone, however, is not sufficient for maximizing the interaction of PHMB with the DNA of *Acanthamoeba* and the effectiveness of the treatment as will be demonstrated hereunder.

Patent application US2007/0140897 A1 [12] describes ophthalmic formulations based on active ingredients containing biguanide (including PHMB) at a pH of 4-6 for the treatment of acanthamoeba keratitis. The only formulations exemplified however are based on alexidine and not on PHMB. In any case, the relevance of the MW and PDI of biguanide-based polymers for interaction with the DNA of the pathogens, or the specific concentrations of use of the biguanide-based polymers in combination with the above-mentioned parameters, are not described.

A stability study of the formulations based on PHMB 0.02% with pH = 4 by Bouattour et al. 2018 [13] reveals the formation of degradation products (BP) already after 90 days. The authors conclude that PHMB-based formulations cannot be stored for more than 60 days in EOS-LDPE bottles at 25°C. The authors of the present invention have now identified a polyhexamethylene biguanide-based formulation for ophthalmic administration that maintains stability and efficacy, at the highest concentrations of active ingredient ranging from 0.04% (w/v) to 0.08%. (w/v) thanks to the control of the molecular weight (MW) and the polydispersion index (PDI) of the PHMB polymer, together with the pH and osmolarity.

Studies have indicated that the use of PHMB with a high molecular weight and polydispersion index together with a specific buffer system that maintains the pH of the PHMB formulation 0.04% -0.08% (w/v) within the range of 5 - 6.5 synergistically creates the optimal conditions for stability at least up to 24 months and for the activity of the active ingredient.

For the reasons indicated above, in fact, the PHMB molecule must be in its protonated form in order to exert its biocidal action mechanism at its best. In the protonated ionized form, PHMB crosses the epithelium of the cornea infected by *Acanthamoeba* and accumulates in the stroma, thanks to the electrostatic interaction with the negative charges of the proteoglycans. It is here that PHMB exerts its biocidal and cysticidal activity with an action mechanism which, according to the most recent scientific research, is expressed through a bond with the DNA through extensive interactions with the phosphate groups of the latter. This bond blocks the replication process of the DNA of the amoeba causing the death of the pathogen [11]. In this sense, the effectiveness of the above-mentioned interaction between PHMB and DNA, and therefore the activity of PHMB itself, are strongly influenced by the stability of the formulation containing the active ingredient. Furthermore, the administration of the formulation according to the invention in a precise graduated dosage regimen in patients affected by *Acanthamoeba* keratitis has proved to be particularly effective in terms of activity against the complete eradication of *Acanthamoeba* cysts.

In this sense, as the formulation object of the invention is a monotherapy, it favours the patient's compliance and adherence to the therapeutic regimen specifically because it avoids the repeated instillation of various products during the day. Furthermore, considering the selectivity and specificity of the product towards the target microorganism, a greater rapidity of action is ensured with a consequent faster resolution of the pathology, avoiding lengthy periods of treatment of 3-6 months.

Again, compared to the multi-drug therapeutic approach composed of powerful antiseptics and broad-spectrum antibiotics currently used, monotherapy (highly selective and specific) with 0.08% PHMB is certainly a safer option for preserving the diversity of ocular microbiota during treatment and therefore for avoiding the appearance of multi-resistant *Staphylococcus epidermidis* strains.

The present invention therefore relates to a liquid formulation based on polyhexamethylene biguanide or its salts at a concentration ranging from 0.04% to 0.08% comprising a buffer system for keeping the pH within the range of 5-6.5 and an isotonizing agent for maintaining an osmolarity within the range of 270-330 mOsm/Kg, wherein the molecular weight of the polyhexamethylene biguanide ranges from 2,300 to 6,000 amu and the polydispersion index of the polymer ranges from 1.5 to 1.9, preferably from 1.7 to 1.8, suitable for ophthalmic administration for use in the treatment of *Acanthamoeba* keratitis or fungal infections.

Said fungal infections are preferably keratitis or keratomycosis mediated by a pathogen selected from the group consisting of *Candida albicans*, *Fusarium solani*, *Aspergillus niger*, *Aspergillus fumigatus*, *Aspergillus flavus,* and *Cladosporium.*

The above-mentioned fungal infections can also be present in conjunction with *Acanthamoeba* infection, in particular *Fusarium solani* and *Cladosporium* infections.

In an alternative embodiment of the invention, said treatment of infectious *Acanthamoeba* keratitis is particularly effective in the case of co-infection with *Pseudomonas aeuriginosa* or *Staphylococcus epidermis.*

The therapeutic use of the formulation of 0.08% PHMB which is active against *Pseudomonas aeruginosa* (data not shown, internal APE test study: ORPHAN DRUG - *in vitro* assay - 1305) proves in fact to be highly performing for a rapid and effective resolution of the pathology in the cases of co-infection indicated above. The polyhexamethylene biguanide can be in the form of an inorganic or organic salt, selected from the group consisting of chloride, bromide, sulfate, phosphate, mesylate, formate, citrate or maleate.

In addition to the need for maintaining a slightly acid pH in order to ensure the maximum protonation state of the PHMB, a further objective of the present invention is to maintain the osmolarity of the formulation at a value comparable to the physiological tear fluid value (which ranges from 290 to 320 mOsm/Kg), preferably by the addition of an ionic isotonizing agent, selected from the group consisting of sodium chloride, potassium chloride, or non-ionic, such as glycerol, mannitol, sorbitol; ionic buffer systems such as phosphate, citrate, bicarbonate or borate buffer or non-ionic buffers such as trometamol, histidine, glycine, HEPES which also provide an osmotic contribution.

The osmotic pressure in fact mainly depends on the ions present in the aqueous layer (Na⁺, K⁺, Cl⁻ and HCO₃).

According to preferred embodiments of the present invention, the isotonizing agent is used in the following concentrations: NaCl (0.01% -0.9%), KCl (0.01% -1.19%), glycerol (0.01 % -2.6%), mannitol (0.01% -5.1%) and sorbitol (0.01% -5.5%).

In a particular embodiment of the liquid formulation based on polyhexamethylene biguanide, the buffer system is an ionic buffer such as a phosphate, citrate, bicarbonate or borate buffer or a non-ionic buffer such as trometamol, histidine, glycine, HEPES, or a mixed system.

The buffer system preferably comprises disodium phosphate dodecahydrate (Na₂HPO₄·12 H₂O) and monobasic sodium phosphate (NaH₂PO₄·H₂O). The disodium phosphate dodecahydrate is preferably used at a concentration within the range of 0.01%-4.5% and the monobasic sodium phosphate is used at a concentration within the range of 0.01% -3.5%.

Alternatively, a citrated phosphate buffer comprising disodium phosphate dodecahydrate (Na₂HPO₄ · 12 H₂O) and citric acid (C₆H₅O₇ ·H₂O) or Tris/HCl or boric acid/borate, can be used.

If the formulation according to the invention is in the form of an ophthalmic gel or a viscous solution, it is preferable to use a phosphate-free buffer, such as, for example, a Tris buffer and/or boric acid or borates.

According to a preferred embodiment of the liquid formulation based on polyhexamethylene biguanide, the pH is 5.8.

In a preferred embodiment of the liquid formulation based on polyhexamethylene biguanide of the invention, the concentration of PHMB is selected from the group consisting of 0.04% (w/v), 0.05% (w/v), 0,06% (w/v), 0.07% (w/v) and 0.08% (w/v), preferably 0.08% (w/v).

The liquid formulation based on polyhexamethylene biguanide according to the invention suitable for ophthalmic administration is a sterile solution which can be in the form of eye drops.

Alternatively, in the presence of a viscosifying agent (e.g. xanthan gum, gellan gum, polyvinyl alcohol, hyaluronic acid, sodium hyaluronate, cellulose derivatives, such as carboxymethyl cellulose or hydroxypropyl cellulose) the polyhexamethylene biguanide-based formulation according to the invention can be in the form of an ophthalmic gel or viscous solution. According to a further preferred embodiment of the invention, the liquid formulation based on polyhexamethylene biguanide according to the invention further comprises a penetration promoter which can be selected from Tween 80 and benzalkonium chloride.

Again according to a preferred embodiment, the liquid formulation based on polyhexamethylene biguanide for use according to the invention can further comprise 0.02% of chlorhexidine, 0.1% of propamidine, 0.1% of desomidine in combination with PHMB. When the PHMB is administered in combination with a second active agent, the administration can be effected sequentially, simultaneously or separately, i.e. in the same eye drop formulation or in separate formulations.

The present invention further relates to the liquid formulation based on polyhexamethylene biguanide according to the invention for the treatment of *Acanthamoeba* keratitis by administering a graduated dosage of 16 drops per day for 5 days, 8 drops per day for 7 days, 6 drops per day for 7 days and 4 drops per day until clinical resolution. Clinical resolution was 88% in an average time of 4 months. This graduated dosage differs from the intensive dosage previously described in the Phase I study conducted by Papa et al. [10] as it intervenes more effectively in the eradication of cysts and therefore in the clinical resolution of *Acanthamoeba* keratitis.

The present invention further relates to a process for the preparation of the liquid formulation based on polyhexamethylene biguanide according to the invention which comprises the following steps:
(i) addition of the buffer system at a concentration ranging from 0.01 to 4.5% (w/v), and the isotonizing agent at a concentration ranging from 0.01% to 5.5% (w/v), in purified water under gentle and continuous stirring; optional addition of a viscosifying agent selected from sodium hyaluronate, xanthan gum, polyvinyl alcohol, carboxymethyl cellulose and hydroxypropyl cellulose;
(ii) addition of polyhexamethylene biguanide having a molecular weight within the range of 2,300-6,000 amu and a polydispersion index within the range of 1.5-1.9, preferably 1.7-1.8, at a concentration ranging from 0.04% to 0.08% (w/v), under mild stirring;
(iii) bringing to volume by the addition of purified water;
(iv) sterilization by filtration or heat.

In the case of a viscous solution, the addition of a viscosifying agent selected from sodium hyaluronate, xanthan gum, polyvinyl alcohol, carboxymethyl cellulose and hydroxypropyl cellulose should also be added after step (i). In this case, in the presence of non-filterable polymers, heat sterilization is used instead of sterilization by filtration.

The solution is then divided into single-dose containers or vials which are then sealed. The single-dose containers can be made of low-density polyethylene (LDPE), every package can contain 5 units of single dose.

In a particular embodiment of the process for preparing the liquid formulation based on polyhexamethylene biguanide, the buffer system for maintaining the pH within the range of 5-6.5 is an ionic buffer such as a phosphate, citrate, bicarbonate, Tris, borate or non-ionic buffer such as trometamol, histidine, glycine, HEPES, or a mixed system. The buffer system is preferably selected from the group consisting of disodium phosphate dodecahydrate/monobasic sodium phosphate, disodium phosphate dodecahydrate/citric acid, Tris/HCl or boric acid/borate.

According to a preferred embodiment, the buffer system is based on disodium phosphate dodecahydrate (Na₂HPO₄ ·12 H₂O) and monobasic sodium phosphate (NaH₂PO₄ · H₂O). The disodium phosphate dodecahydrate is preferably used at a concentration within the range of 0.01% -4.5% (w/v), and monobasic sodium phosphate is used at a concentration within the range of 0.01% -3.5% (w/v).

The isotonizing agent for keeping an osmolarity within the range of 270-330 mOsm/Kg is preferably sodium chloride.

The preparation process of the formulation was developed for also ensuring that both the initial molecular weight and the polydispersion index of the active ingredient PHMB are kept in the finished product within the range of 2,300-6,000 amu and 1.5-1.9, preferably 1.7-1.8, respectively. These parameters have proved to be responsible for the effectiveness of the finished product.

Finally, the present invention relates to therapeutic eye lenses loaded or derivatized with the liquid formulation based on polyhexamethylene biguanide according to the invention for the controlled release of the active ingredient for the treatment of *Acanthamoeba* keratitis or co-infections by *Pseudomonas aeuriginosa.* This embodiment allows for the continuous release of the formulation for prolonged periods and with a further gain in terms of compliance and adherence to therapy on the part of the patient.

The present invention will now be described for illustrative but non-limiting purposes, according to a preferred embodiment with particular reference to the attached figures, in which:
- Figure 1 shows the structure of the repeating unit of hexamethylene biguanide in PHMB;
- Figure 2 shows a flow chart of the preparation process of the PHMB-based formulations;
- Figure 3 illustrates the chromatogram obtained by gel-permeation chromatography of the PHMB in API.
- Figure 4 illustrates the chromatogram obtained by gel-permeation chromatography of the PHMB in the finished product; from a comparison with the chromatogram of Figure 2 referring to the raw material, it is evident how the production process is capable of keeping the molecular-weight distribution of the raw material unchanged, up to the finished product.
- Figure 5 shows the comparison between the stability data of the 0.08% PHMB-based formulation at pH 7.4 and pH 5.8.

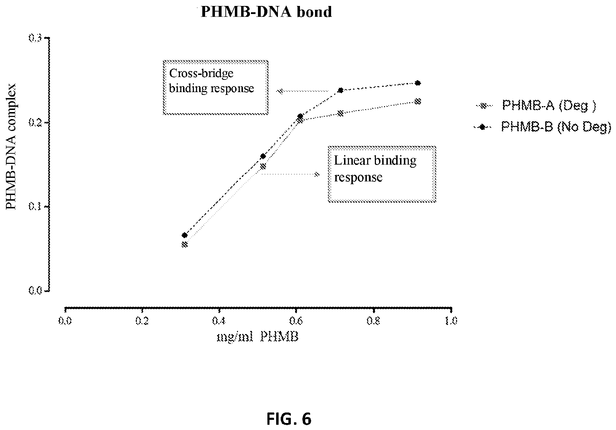

- Figure 6 shows a comparison between the PHMB-DNA bond in relation to the molecular weight. From the trend of the graph it can be appreciated that using PHMB with a high molecular weight the polymer-DNA bond is more effective as one or more nucleic acid molecules are involved. The high-molecular-weight polymer is capable of forming cross-bridge links between different nucleic acid molecules forming a network of bonds (line with circles). Vice versa, the line with squares reflects the establishment of a linear type bond between PHMB and DNA, in which the polymer characterized by a lower molecular weight cannot show an aptitude for forming cross-bridge bonds but establishes a bond in which only one nucleic acid molecule is involved.

- Figure 7 (A) shows the results of the stability study of 0.08% PHMB formulations using PHMB batches with different molecular weights (MW) and polydispersion indexes (PDI), expressed as % recovery (w/v) of PHMB at T0 and after 12 months (T12).
- Figure 7 (B) shows the results of the stability study of a 0.08% PHMB formulation, using a PHMB batch characterized by a MW = 2,641 amu and a PDI = 1.6. The study was carried out under three different temperature conditions: long term stability 25°C, RH 40%; intermediate stability 30°C, RH 65%; accelerated stability 40°C RH 25%. Stability results expressed as % recovery (w/v) of PHMB at T0 and after 3 months (T3) are indicated.

The following non-limiting examples are now provided for a better illustration of the invention, wherein various PHMB-based formulations were tested and compared, evaluating the impact on the stability of some formulation parameters such as pH, osmolarity, molecular weight and polydispersion index of the active ingredient PHMB.

### EXAMPLE 1: Preparation process of PHMB-based formulations

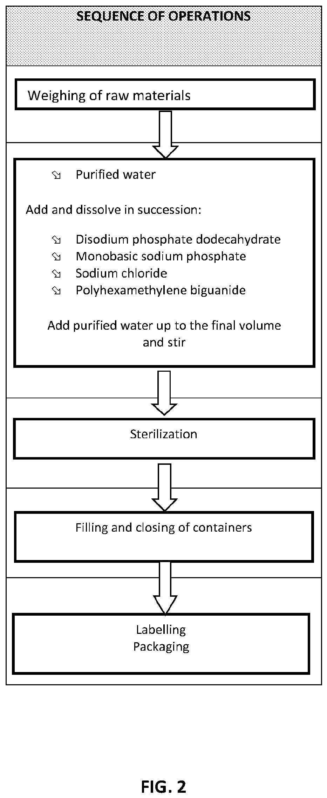

The process for preparing the formulation according to the invention is illustrated in the diagram of Figure 2.

Disodium phosphate dodecahydrate (Na₂HPO₄ ·12 H₂O), monobasic sodium phosphate (NaH₂PO₄ · H₂O) and sodium chloride are added in succession and dissolved in a volume of purified water under continuous gentle stirring.

The stirring step is critical for maintaining the initial molecular weight and the polydispersion index of PHMB and must be effected avoiding the formation of foam or vortexes in the solution. A stir bar or wheel to be immersed in the solution for stirring is preferably used. Finally, PHMB (0.08%) is added.

The solution is brought to volume (100 ml) by adding a sufficient quantity of purified water and under continuous stirring. The pH and appearance of the solution are then controlled.

Sterilization is effected by filtration through a 0.2 µM filter. The nature of the filter membrane and the dynamics (pressure, flow) are selected so as to prevent the product from being forced through the membrane to avoid depolymerization or breakage of the polymer chain.

After filtration, the integrity of the 0.2 µm filter is controlled by means of an electronic control system.

Finally, the sterile solution is used for filling single-dose containers using the Blow/Fill/Seal (B/F/S) technology under aseptic conditions.

This automated technology allows a vial to be filled and sealed, under aseptic conditions with solution volumes ranging from 0.1 to 1,000 cm³.

The PHMB solution appears as a clear colourless or pale yellow liquid.

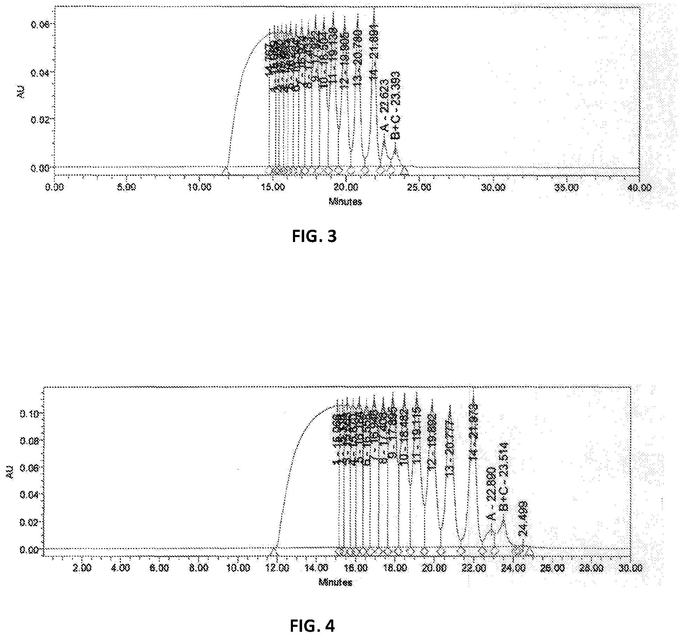

At the end of the production process, the characterization of the finished product in its main components is effected, including any impurities with suitable chromatographic methods. The comparison between the PHMB chromatogram in the API (Figure 3) and the PHMB chromatogram following the preparation process of the formulation (Figure 4), shows that the molecular-weight distribution of the PHMB is unaltered.

### EXAMPLE 2: Comparison studies on the stability of PHMB-based formulations

The PHMB-based formulations produced are sterile solutions whose relevant physicochemical parameters are pH and osmolarity.

As detailed, the choice of a slightly acidic pH is governed by the necessity of obtaining a stable formulation that cannot be obtained at a pH around neutrality.

For this purpose, various formulations based on PHMB 0.08% at different pH values (5.8, 6.2, 7.4) were developed, subsequently verifying their stability at 25°C, 40°C and 60°C. For the preparation of the formulation based on PHMB 0.08%, pH 5.8 the following ingredients were weighed:

**Table 1**

| **Ingredient** | **Function** | **% (w/v)** |
|---|---|---|
| NaH₂PO₄ · H₂O | buffer | 0.827 g |
| Na₂HPO₄ ·12 H₂O | buffer | 0.240 g |
| NaCl | isotonizing agent | 0.520 g |
| PHMB | active ingredient | 0.08 g |
| Sterile water | solvent | complement to 100 ml |

Table 2 hereunder indicates the physico-chemical characteristics of the final formulation at pH 5.8 and during the phases of the preparation process of the same.

**Table 2**

| | **pH** | **Osmolarity (mOsm/Kg)** | **Active UV spectrum** | **Recovery (UV quantitative)** |
|---|---|---|---|---|
| Buffer solution (Before adding PHMB) | - | 289 | - | - |
| Buffer with PHMB (before sterilization) | 5.8 | 290 | corresponds | - |
| Formulation sterilized by filtration | 5.8 | 297 | corresponds | 100.4 |

For the preparation of the formulation based on PHMB 0.08%, pH 6.2 the following ingredients were weighed following the same process described above:

**Table 3**

| **Ingredient** | **Function** | **% (w/v)** |
|---|---|---|
| NaH₂PO₄ · H₂O | buffer | 0.735 g |
| Na₂HPO₄ ·12 H₂O | buffer | 0.480 g |
| NaCl | isotonizing agent | 0.510 g |
| PHMB | active ingredient | 0.08 g |
| Sterile water | solvent | complement to 100 ml |

Table 4 hereunder indicates the physico-chemical characteristics of the final formulation at pH 6.2 and during the phases of the preparation process of the same.

**Table 4**

| | **pH** | **Osmolarity (mOsm/Kg)** | **Active UV spectrum** | **Recovery (UV quantitative)** |
|---|---|---|---|---|
| Buffer solution (Before adding PHMB) | 6.05 | 291 | - | - |
| Buffer with PHMB (before sterilization) | 6.07 | 295 | corresponds | - |
| Sterilized formulation by filtration | 6.20 | 287 | corresponds | 100.9 |

For the preparation of the formulation based on 0.08% PHMB, pH 7.4, the following ingredients were weighed:

**Table 5**

| **Ingredient** | **Function** | **% (w/v)** |
|---|---|---|
| NaH₂PO₄ · H₂O | buffer | 1.91 g |
| Na₂HPO₄ ·12 H₂O | buffer | 0.19 g |
| NaCl | isotonizing agent | 0.44 g |
| PHMB | active ingredient | 0.08 g |
| Sterile water | solvent | complement to 100 ml |

Table 6 hereunder indicates the physico-chemical characteristics of the final formulation at pH 7.4 and during the phases of the preparation process of the same.

**Table 6**

| | **pH** | **Osmolarity (mOsm/Kg)** | **Active UV spectrum** |
|---|---|---|---|
| Buffer solution (Before adding PHMB) | - | - | - |
| Buffer with PHMB | 7.45 | 286 | corresponds |
| (before sterilization) | | | |
| Formulation sterilized by filtration | 7.45 | 286 | corresponds |
| Formulation sterilized in an autoclave | 7.33 | 295 | corresponds |
| Filtered formulation, after 2 weeks at 25°C | 7.36 | 293 | corresponds |

Comparisons were then made on the stability of 0.08% PHMB-based formulations in a single dose container at a pH of 5.8 and 7.4 at 25°C (long term), 40°C (accelerated) and 60°C (stress conditions).

The results of the stability analyses are indicated hereunder:

### • Stability data:

- 0.08% PHMB formulation, pH 5.8 at 25°C ± 2°C and relative humidity 40% ± 5 (long term)

**Table 7**

| | **Limits** | | | **Weeks** | | | | | | |
|---|---|---|---|---|---|---|---|---|---|---|
| **Analyses** | **Meas. Unit** | **Min** | **Max** | **0** | **13** | **26** | **39** | **52** | **65** | **104** |
| Appearance | Transparent, free of foreign particles | | | corr.* | corr. | corr. | corr. | corr. | corr. | corr. |
| Colour | Colourless | | | corr. | corr. | corr. | corr. | corr. | corr. | corr. |
| pH | | 5.3 | 6.3 | 5.7 | 5.6 | 5.8 | 5.8 | 5.8 | 5.8 | 5.8 |
| Osmolarity | mOsm/Kg | 270 | 330 | 290 | 274 | 281 | 276 | 293 | 277 | 276 |
| Biguanide (HPLC) | Corresponds to standard | | | corr. | corr. | corr. | corr. | corr. | corr. | corr. |
| Biguanide test (UV) | % L.S. | 90 | 110 | 97.6 | 97.8 | 99.7 | 96.2 | 98.8 | 92.5 | 94.6 |
| Impurity A | % a.s. | | 5.0 | 0.8 | 1.0 | 1.0 | 1.8 | 0.8 | 1.0 | 0.9 |
| Impurities | % a.s. | | 5.0 | 0.9 | 0.9 | 1.2 | 2.4 | 1.1 | 1.4 | 1.1 |
| B+C | | | | | | | | | | |
| Water loss | % | | 5.0 | | 0.0 | 0.1 | 0.1 | 0.1 | 0.1 | 0.2 |
| Sterility | According to European pharmacopoeia | | | sterile | | | | | | sterile |

| | | | | | | | | | | |
|---|---|---|---|---|---|---|---|---|---|---|
| *corr.: corresponding | | | | | | | | | | |

- 0.08% PHMB formulation, pH 5.8 at 40°C± 2° C and relative humidity 25% (accelerated)

**Table 8**

| | **Limits** | | | **Weeks** | | |
|---|---|---|---|---|---|---|
| **Analysis** | **Meas. Unit** | **Min.** | **Max.** | **0** | **13** | **26** |
| Appearance | Transparent, free of foreign particles | | | corr. | corr. | corr. |
| Colour | Colourless | | | corr. | corr. | corr. |
| pH | | 5.3 | 6.3 | 5.7 | 5.6 | 5.8 |
| Osmolarity | mOsm/Kg | 270 | 330 | 290 | 274 | 276 |
| Biguanide (HPLC) | Corresponding to the standard | | | corr. | | corr. |
| Biguanide test (UV) | % L.S. | 90 | 110 | 97.6 | 96.7 | 99.2 |
| Impurity A | % a.s. | | 5.0 | 0.8 | 1.0 | 0.9 |
| Impur. B+ C | % a.s. | | 5.0 | 0.9 | 1.0 | 1.3 |
| Water loss | % | | 5.0 | | 0.1 | 0.2 |
| Sterility | According to European Pharmacopoeia | | | sterile | | |

| | | | | | | |
|---|---|---|---|---|---|---|
| *corr.: corresponding | | | | | | |

0.08% PHMB formulation, pH 7.4 at 60°C ± 2°C and relative humidity 20% (stress conditions)

**Table 9**

| | **Limits** | | | **Weeks** | | |
|---|---|---|---|---|---|---|
| **Analysis** | **Meas.units** | **Min.** | **Max.** | **0** | **1** | **2** |
| Appearance | Transparent, free of foreign particles | | | corr. | corr. | corr. |
| Colour | Colourless | | | corr. | corr. | corr. |
| pH | | 7.0 | 7.8 | 7.3 | 7.2 | 7.3 |
| Osmolarity | mOsm/Kg | 270 | 330 | 276 | 294 | 299 |
| Biguanide (ABS ratio 235/220nm) | | | | 1.5 | 1.4 | 1.3 |
| Sterility | According to European Pharmacopoeia | | | sterile | | sterile |

| | | | | | | |
|---|---|---|---|---|---|---|
| *corr.: corresponding | | | | | | |

PHMB 0.08% formulation, pH 5.8 at 60°C ± 2°C and relative humidity 20% (stress conditions)

**Table 10**

| | **Limits** | | | **Weeks** | | |
|---|---|---|---|---|---|---|
| **Analysis** | **Meas.units** | **Min.** | **Max.** | **0** | **1** | **2** |
| Appearance | Transparent, free of foreign particles | | | corr. | corr. | corr. |
| Colour | Colourless | | | corr. | corr. | corr. |
| pH | | 5.3 | 6.3 | 5.8 | 5.6 | 5.7 |
| Osmolarity | mOsm/Kg | 270 | 330 | 289 | 296 | 302 |
| Biguanide (ABS ratio 235/220nm) | | | | 1.4 | 1.5 | 1.5 |
| Sterility | According to European Pharmacopoeia | | | sterile | | sterile |

| | | | | | | |
|---|---|---|---|---|---|---|
| *corr.: corresponding | | | | | | |

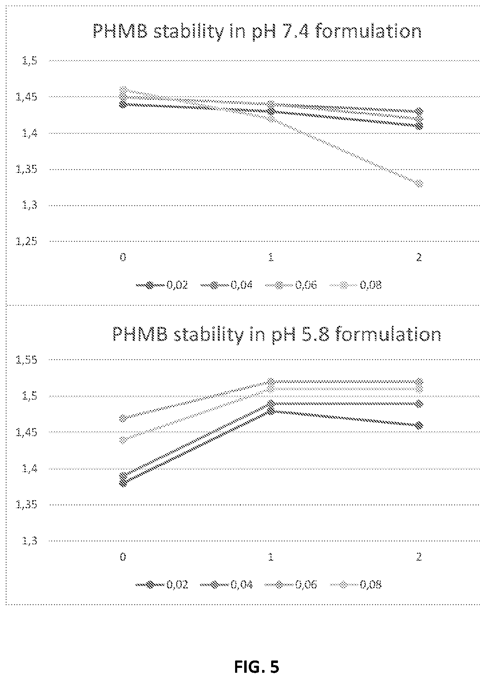

The results shown in Figure 5 show that the stability of the PHMB-based formulation obtained by maintaining a pH within the range of 5-6.5 is not achieved with a pH around neutral (pH 7.4).

### EXAMPLE 3: Study of the PHMB-DNA bond

A study on the PHMB-DNA bond was carried out in order to evaluate the equivalence between low-molecular-weight PHMB and high-molecular-weight PHMB.

The binding capacity of PHMB to DNA can be considered as being independent of the specific type of DNA studied as indicated in literature [8], whereas the binding capacity and effectiveness to DNA depend on the molecular weight and polydispersion index of the PHMB polymer. When the polymer has a molecular weight ranging from 2,300 to 6,000 amu and a polydispersion index within the range of 1.5-1.9, the binding with DNA is more effective, probably because the length of the polymer is more suitable for the formation of cross-bridge bonds involving multiple nucleic acid molecules according to the mechanism proposed by Allen et al. [8]. Vice versa, when the polymer is characterized by a low molecular weight the bond will be linear and will involve a single nucleic acid molecule.

A comparative study was then conducted with different PHMB polymer samples in order to evaluate the binding capacity to nucleic acid molecules. The PHMB (sample A) was degraded by acid and high-temperature treatment for 5 hours to obtain low-molecular-weight PHMB polymers. The PHMB (sample B) did not receive any type of treatment and is characterized by a molecular weight that falls within the range claimed.

The capacity of both samples of binding nucleic acid molecules was then evaluated. The experimental method proposed herein is based on the interaction between an aqueous solution of PHMB and an aqueous solution of DNA. The quantity of the PHMB-DNA complex was measured as the difference between the initial quantity of both species in solution and the residual quantity after the end of the reaction. The quantity of both species in solution is monitored by spectrophotometric measurements at 2 different wavelengths, 260 nm for DNA and 235 nm for PHMB, respectively.

In conclusion, the data show an increased capacity of high-molecular-weight PHMB in interacting with DNA through the formation of cross-bridge bonds with multiple nucleic acid molecules and therefore a consequent greater efficacy in the treatment of infectious *Acanthamoeba* keratitis or of co-infections from *Pseudomonas aeruginosa.* The type of bond between the PHMB polymer and DNA that is created is much more effective, as shown in the graph in Figure 6.

### EXAMPLE 4: Stability study in relation to the molecular weight and the polydispersion index of PHMB

A comparative study was carried out on three different PHMB-based starting batches characterized by different molecular weights and polydispersion indexes (PDI):
- batch L-17GR185627, MW = 2517 amu, PDI = 1.73,
- batch F-693/LU/101, MW = 1170 amu, PDI = 1.93;
- batch F-693/LU/118, MW = 440 amu, PDI = 2.14.

For each lot a 0.08% PHMB formulation was prepared characterized by pH = 5.8, osmolarity 0.280 osmol/Kg.

The stability of the PHMB formulation was then evaluated at T0 and after 12 months (T = 12) at a temperature of 25°C ± 2°C, RH 40 ± 5%. The parameters considered are the following:
- Appearance
- Recovery% (w/v) of the active ingredient

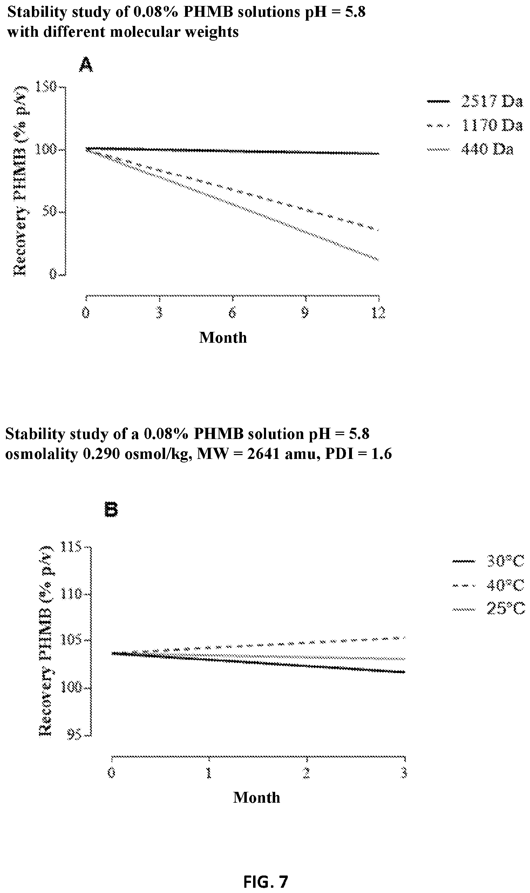

The results are indicated in the following Table 11 and in the graph of Figure 7A.

**Table 11**

| **Batch** | **MW (Da)** | **PDI** | **Test** | **Months** | |
|---|---|---|---|---|---|
| | | | | **T= 0** | **T= 12** |
| L-17GR185627 | 2517 | 1.73 | Appearance | Acceptable | Acceptable |
| | | | Recovery % PHMB (UV) | 101.5 | 97.1 |
| F-693/LU/101 | 1170 | 1.93 | Appearance | Acceptable | Not acceptable |
| | | | Recovery % PHMB (UV) | 100 | 35.8 |
| F-693/LU/118 | 440 | 2.14 | Appearance | Acceptable | Non acceptable |
| | | | Recovery % PHMB (UV) | 100 | 12.2 |

The stability study confirms that the most stable 0.08% PHMB formulation is that characterized by the PHMB with the highest molecular weight, i.e. batch L-17GR185627, MW = 2517 amu, pH = 5.8 and with a PDI value of 1.73. No significant degradation products (BP) were observed throughout the duration of the stability study.

0.08% PHMB solutions characterized by a low molecular weight (batch F-693/LU/101, MW = 1170 amu - batch F-693/LU/118, MW = 440 amu) indicate a reduced recovery % of PHMB after 12 months and therefore instability of the formulation and reduced effectiveness.

It is therefore evident that the parameters of the molecular weight and polydispersion index of PHMB together with the pH of the solution contribute synergistically to providing a stable and effective formulation.

Furthermore, a stability study was carried out on 0.08% PHMB solutions, at pH 5.8 and osmolality 0.290 osmol/Kg, characterized by a MW = 2641 amu and a PDI = 1.6. The study was carried out under three different temperatures conditions: Long term stability25°C, RH 40%; Intermediate stability 30°C, RH 65%; Accelerated stability 40°C RH 25%. The stability results are indicated hereunder, and in Figure 7 (B):

**Table 12**

| **Conditions** | **Test** | **Months** | |
|---|---|---|---|
| | | **T= 0** | **T= 3** |
| 25°C, RH 40% | Appearance | Acceptable | Acceptable |
| | Recovery % PHMB (UV) | 103.7 | 103.1 |
| 30°C, RH 65%; | Appearance | Acceptable | Acceptable |
| | Recovery % PHMB (UV) | 103.7 | 101.7 |
| 40°C RH 25% | Appearance | Acceptable | Acceptable |
| | Recovery % PHMB (UV) | 103.7 | 105.3 |

The results of the stability tests show that the product is stable under all the temperature conditions investigated, as the recovery % of PHMB falls within the predetermined specifications limit , 90 - 110% (w/v). The other chemical-physical parameters investigated (data not shown), such as pH, osmolality and recovery assay of the impurities, remain stable and within the specified specification limits, under all of the three climatic conditions tested.

### EXAMPLE 5: Clinical study

A randomized, multicentre, double-blind, parallel-group Phase 3 study is described in detail hereunder, for evaluating the efficacy, safety and tolerability of the 0.08% PHMB formulation of the invention compared to a conventional combination therapy 0.02% PHMB + 0.1% propamidine in male and female adult subjects suffering from *Acanthamoeba* keratitis.

The study is conceived as a superiority study in accordance with EMA requirements (CPMP/EWP/482/99). The study comprises a screening visit for inclusion, a treatment period that includes short outpatient visits and follow-up visits.

A total of 130 subjects with *Acanthamoeba* keratitis were assigned to the following two treatment groups in a 1:1 ratio.
Group 1: 0.08% PHMB + placebo
Group 2: 0.02% PHMB + 0.1% combination therapy.

### Patients:

The study was conducted in male and female subjects suffering from *Acanthamoeba* keratitis, ≥12 years of age, inclusive.

### Objective:

The primary objective of the study is the comparison of the Clinical Resolution Rate (CRR) 12 months after randomization (CRR_12) between the 0.08% PHMB + placebo formulation and the combination therapy 0.02% PHMB + 0.1% propamidine, evaluating the difference between CRR_12 in relation to the degree of uncertainty, and testing the therapeutic superiority or non-inferiority of monotherapy with 0.08% PHMB

A further objective of the study is to obtain information on the safety of the ophthalmic formulation based on 0.08% PHMB according to the invention.

### Hypothesis:

The primary hypothesis to be tested is that the CRR12 of the subjects treated with monotherapy with the 0.08% PHMB formulation is higher or not inferior, lower by a still acceptable margin (Δ), with respect to the CRR12 of the combination therapy 0.02% PHMB + 0.1% propamidine, administered according to the treatment protocol indicated hereunder.

The secondary hypotheses are:
- the undesirable effects relating to toxicity are lower with the 0.08% PHMB monotherapy compared to the combination therapy;
- *time to cure* is lower in subjects receiving the 0.08% PHMB monotherapy compared to the combination therapy.

The clinical resolution obtained within 12 months of starting treatment is 67%, as indicated in the sponsor's retrospective study (Study 038/SI).

### Concurrent and previous treatments

The subjects are authorized to take or have taken the following drugs:
Antibiotics: Topical moxifloxacin is allowed for the treatment of concomitant bacterial infections. It is not permitted however as a prophylactic antibiotic for the treatment of patients with corneal ulcers; PHMB is already a broad-spectrum antibacterial and an additional antibacterial is not needed. Topical moxifloxacin is not permitted for use as a prophylactic antibiotic in patients with corneal ulcers; PHMB is a good broad-spectrum anti-bacterial and an additional antibacterial is not needed for this.
Antiviral and antifungal drugs: The use of these drugs is not allowed during the study. Any intake of these drugs at the onset of the study must be discontinued.
Anti-inflammatory drugs: For subjects under steroid treatment at the onset of the study (who are already taking local steroids, e.g. for a misdiagnosis of HSV keratitis or as an adjuvant treatment for bacterial keratitis) the following options exist:
   a. Interruption, maintenance or reduction of steroid doses. Dexamethasone (0.1% or 0.15%) is the only local steroid allowed in this trial. Patients using other topical steroids at the time of the start of the study must change the frequency of administration. Diclofenac is the only oral NSAID allowed in the trial and is added in the appropriate doses (75 mg - 150 mg per day, divided into 2-3 doses).
   b. Subjects using NSAIDS or cyclosporine at the time of the start of the clinical study must stop treatment after randomization.
   c. Subjects who are not using local steroids at the time of entry into the study can start them together with oral NSAIDs (recommended diclofenac; 75 mg - 150 mg per day, divided into 2-3 doses) during the study as specified in the protocol.

Other topical therapeutic treatments permitted: lubricants, mydriatics (cyclopentolate, homatropine or atropine) and anti-glaucoma drugs.

### Dosage

The dosage applied in the clinical efficacy study is as follows:
administration of a graduated dosage of 16 drops per day for 5 days, 8 drops per day for 7 days, 6 drops per day for 7 days and 4 drops per day until clinical resolution.

### Treatment assignment:

Protocol for bilateral pathology: if both eyes are affected only one of the two (the right unless there is a difference in severity such as to require treatment of the more compromised eye) will be treated according to the dosage of the treatment and considered for the study. The other eye will be treated according to standard clinical practice.

Table 13 below summarizes the demographics and distribution of patients participating in the study.

**Table 13**

| | **PHMB 0.08%** + **placebo** | **PHMB 0.02% + 0.1% Propamidine** | **Total** |
|---|---|---|---|
| Screened | 69 | 66 | 135 |
| Screening failures | 0 | 0 | 0 |
| Randomized | 69 | 66 | 135 |
| Did not receive the study drug¹ | 0 | 1 (1.5%) | 1 (0.7%) |
| Safety analysis set¹ | 69 (100%) | 65 (98.5%) | 134 (99.3%) |
| Study completion ² | 58 (84.1%) | 57 (87.7%) | 115 (85.8%) |
| Premature withdrawals from the study ² | 11 (15.9%) | 7 (10.8%) | 18 (13.4%) |
| did not receive the study drug | 0 | 1 (100.0%) | 1 (100.0%) |
| Full analysis set¹ | 66 (95.7%) | 61 (92.4%) | 127 (94.1%) |
| Study completion ² | 56 (84.8%) | 53 (86.9%) | 109 (85.8%) |
| Premature withdrawals from the study ² | 10 (15.2%) | 7 (11.5%) | 17 (13.4%) |
| DX not confirmed | 3 (100%) | 4 (80%) | 7 (87.5%) |
| Absence of primary efficacy evaluation | 0 | 1 (20%) | 1 (12.5%) |
| Protocol analysis set¹ | 62 (89.9%) | 57 (86.4%) | 119 (88.1%) |
| Study completion² | 54 (87.1%) | 50 (87.7%) | 104 (87.4%) |
| Premature withdrawals from the study ² | 8 (12.9%) | 6 (10.5%) | 14 (11.8%) |
| Maximum PD | 4 (57.1%) | 4 (44.4%) | 8 (50%) |
| Not FAS | 3 (42.9%) | 5 (55.6%) | 8 (50%) |
| ¹ Percentages based on the number of randomized patients within each treatment group | | | |
| ² Percentages based on the number of subjects included in the analysis set | | | |

Table 14 shows the 12-month clinical resolution rate. Patients are divided into patients with and without prior steroid treatment.

**Table 14**

| **Clinical resolution rate 12 months** | | | **PHMB 0.08% PHMB placebo (N=66)** | **PHMB** + **0.02%+ 0,1% Propamidine (N=61)** | **Total (N=127)** |
|---|---|---|---|---|---|
| Prior steroid-treated patients* | Unresolved | 7 (22.6%) | 2 (10%) | 9 (17.6%) | |
| | Resolution | 24 (77.4%) | 18 (90%) | 42 (82.4%) | |
| Patients without prior steroid treatment | Unresolved | 3 (8.6%) | 5 (12.2%) | 8 (10.5%) | |
| | Resolution | 32 (91.4%) | 36 (87.8%) | 68 (89.5%) | |
| * Any use of any of these drugs prior to therapy: | | | | | |
| Corticosteroids and anti-infective drugs in combination, corticosteroids | | | | | |
| The percentages are based on the number of subjects within each subgroup and treatment | | | | | |

### Results:

### Primary efficacy: 12-month clinical resolution rate

The results of the clinical study reveal the non-inferiority of 0.08% PHMB monotherapy compared to the combination therapy 0.02% PHMB + Propamidine 0.1% in terms of clinical resolution rate at 12 months (CRR_12)
* CRR_12 for 0.08% PHMB monotherapy is equal to 87.1%
* CRR_12 for 0.02% PHMB + Propamidine 0.1% is equal to 89.5%

Monotherapy is always preferred to combination therapy and the results do not show any statistically significant differences.

The percentage of subjects previously treated with corticosteroid-based therapies are indicated hereunder:
- 22.6% for 0.08% PHMB monotherapy
- 10% for 0.02% PHMB + Propamidine 0.1% combination therapy

The CRR12 clinical resolution rate for patients with and without prior steroid treatment are indicated hereunder:
* CRR_12 for monotherapy 0.08% PHMB is equal to 91.4%
* CRR_12 for 0.02% PHMB + Propamidine 0.1% is equal to 87.8%

The CRR_12 obtained with monotherapy is in both cases unexpected, as the combination therapy followed in normal clinical practice provides a CRR_12 of approximately 63-67%. The clinical study therefore not only demonstrates that monotherapy under the formulation conditions described (MW, PDI and PHMB polymer concentration 0.04% -0.008%, pH and osmolality) is equivalent to the combination therapy, but in both cases the results obtained are better than those previously described in clinical practice (retrospective study 083/SI).

### Secondary efficacy: cure time

The cure times associated with the treatment are:
* 0.08% PHMB group: 138.3 days* (range 32-365 days)
* 0.02% PHMB group + Propamidine 0.1%: 117.1* days
   (range 54-214 days)
*average value

0.08% PHMB shows a cure time within a wider range. A longer cure time is generally associated with a monotherapy treatment with respect to a combination therapy, but unexpectedly the lower limit of the cure time range for monotherapy shows a resolution at 32 days. The results do not show any statistical difference.

Table 15 shows the results for the time-to-care of patients who have been treated

**Table 15**

| **Treatment time (days)** | **PHMB 0.08%+ placebo (N=56)** | **PHMB 0.02% + 0,1% Propami-dine (N=54)** |
|---|---|---|
| Visit at the end of the study n/nmiss | 56/0 | 54/0 |
| Average (DS) | 138.3 (67,3) | 117.1 (47) |
| Mean | 125 | 112 |
| Q1, Q3 | 93. 152 | 85. 151 |
| Min, Max | 32. 365 | 54. 214 |
| p-value for the difference between treatment groups¹ | 0.0934 | |
| n/nmiss = number of subjects with evaluated/missing data | | |
| Q1 = first quartile, Q3 = third quartile, SD = standard deviation ¹Mann Whitney U test. | | |

### BIBLIOGRAPHY

[1] Kilvington S, Larkin DF. Acanthamoeba adherence to contact lenses and removal by cleaning agents. Eye 1990;4:589-593.
[2] Sharma R., et al. Coinfection with Acanthamoeba and Pseudomonas in contact lens-associated keratitis. Optom Vis Sci. 2013; 90(2): e53-5
[3] Singh A., et al. Acanthamoeba Keratitis Versus Mixed Acanthamoeba and Bacterial Keratitis: Comparison of Clinical and Microbiological Profiles. Cornea. 2020; 39:1112-1116.
[4] Nakagawa H., et al. Number of Bacteria and Time of Coincubation With Bacteria Required for the Development of Acanthamoeba Keratitis. Cornea. 2017; 36:353-357.
[5] Larkin D. F., et al., Treatment of Acanthamoeba keratitis with polyhexamethylene biguanide, Ophthalmology, 99 (1992) 185.
[6] Dave S.B., Changes in ocular flora in eyes exposed to ophthalmic antibiotics. Ophthalmology. 2013;120:937-41.
[7] Ian J. et al., Effects of Polyhexamethylene Biguanide and Polyquaternium-lon Phospholipid Bilayer Structure and Dynamics, J. Phys. Chem. B 2015, 119, 10531-10542.
[8] M. J. Allen, et al. White Cooperativity in the binding of the cationic biocide polyhexamethylene biguanide to nucleic acids. Biochemical and Biophysical Research Communications 318 (2004) 397-404.
[9] K. Chindera, et al. The antimicrobial polymer PHMB enters cells and selectively condenses bacterial chromosomes. Scientific Reports 6:23121.
[10] Papa V. et al. Ocular safety and tolerability of high dose PHMB (Polyhexanide) in healthy volunteers. ARVO, 2017.
[11] Sowlati-Hashjin S., et al. Insights into the Polyhexamethylene Biguanide (PHMB) Mechanism of Action on Bacterial Membrane and DNA: A Molecular Dynamics Study. J Phys Chem B. 2020;124:4487-4497.
[12] Domanda di brevetto US2007/0140897 A1.
[13] Y. Bouattour et al. Stability of an ophtalmic formulation of polyhexamethylene biguanide in gamma-sterilized and ethylene oxide sterilized low density polypropylene multidose eyedroppers. PEERJ, vol. 6 pag. e4549 (2018).

## Claims

1. A liquid formulation based on polyhexamethylene biguanide comprising polyhexamethylene biguanide at a concentration within the range of 0,04%-0,08% (w/v), a buffer system for keeping the pH within the range of 5-6,5, an isotonizing agent for keeping the osmolarity within the range of 270-330 mOsm/Kg, **characterized by** a molecular weight of polyhexamethylene biguanide within the range of 2,300-6,000 amu and a polydispersion index of the polymer within the range of 1.5-1.9, suitable for ophthalmic administration for use in the treatment of *Acanthamoeba* keratitis and/or fungal infections.

2. The liquid formulation based on polyhexamethylene biguanide for use according to claim 1, for the treatment of co-infections by *Acanthamoeba* and *Pseudomonas aeruginos* or *Staphylococcus epidermis.*

3. The liquid formulation based on polyhexamethylene biguanide for use according to claims 1-2, wherein the buffer system is selected from the group comprising phosphate, citrate, bicarbonate, borate, Tris, glycerol, mannitol, sorbitol, trometamol, histidine, glycine, HEPES buffer, or a mixed system.

4. The liquid formulation based on polyhexamethylene biguanide for use according to claim 3, wherein said buffer system is selected from the group consisting of disodium phosphate dodecahydrate/monobasic sodium phosphate, disodium phosphate dodecahydrate/citric acid, Tris/HCl or boric acid/borate.

5. The liquid formulation based on polyhexamethylene biguanide for use according to any of claims 1-4, wherein the isotonizing agent is sodium chloride or potassium chloride.

6. The liquid formulation based on polyhexamethylene biguanide for use according to any of claims 1-5, **characterized by** a polydispersity index of the polymer within the range of 1.7-1.8.

7. The liquid formulation based on polyhexamethylene biguanide for use according to any of claims 1-6, having a pH of 5.8.

8. The liquid formulation based on polyhexamethylene biguanide for use according to any of claims 1-7, in the form of a collyrium, eye drops, ophthalmic gel or viscose solutions.

9. The liquid formulation based on polyhexamethylene biguanide for use according claim 8, in the form of an ophthalmic gel or a viscose solution, further comprising a viscosifying agent selected from the group consisting of Xanthan gum, gellan gum, polyvinyl alcohol, hyaluronic acid, sodium hyaluronate, carboxymethylcellulose and hydroxypropylcellulose.

10. The liquid formulation based on polyhexamethylene biguanide for use according to any of claims 1-9, further comprising a penetration enhancer selected from Tween 80 and benzalkonium chloride.

11. The liquid formulation based on polyhexamethylene biguanide for use according to any of claims 1-10, further comprising 0.02% chlorhexidine, 0.1% propamidine or 0.1% desomidine.

12. The liquid formulation based on polyhexamethylene biguanide for use according to any of claims 1-11, by ophthalmic administration of a graduated dosage of 16 drops/day for 5 days, 8 drops/day for 7 days, 6 drops/day for 7 days and 4 drops/day until clinical resolution.

13. A process for the preparation of a liquid formulation based on polyhexamethylene biguanide according to any of claims 1-12, comprising the following steps:
(i) addition of the buffer system at a concentration ranging from 0,01% to 4,5% (w/v) and of the isotonizing agent at a concentration ranging from 0,01% and 5,5% (w/v) in purified water under gentle and continuous stirring; optional addition of a viscosifying agent selected from sodium hyaluronate, xanthan gum, polyvinyl alcohol, carboxymethylcellulose and hydroxypropylcellulose;
(ii) addition of polyhexamethylene biguanide having a molecular weight within the range of 2,300-6,000 amu and a polydispersion index within the range of 1.5-1.9, preferably 1.7-1.8, at a concentration within the range of 0.04%-0.08% (w/v) under gentle stirring;
(iii) bringing to volume by the addition of purified water;
(iv) sterilization by filtration or heat.

14. The process for the preparation of a liquid formulation based on polyhexamethylene biguanide according to claim 13, wherein said buffer system is selected from the group comprising phosphate, citrate, bicarbonate, borate, Tris, glycerol, mannitol, sorbitol, trometamol, histidine, glycine, HEPES buffer, or a mixed system.

15. The process for the preparation of a liquid formulation based on polyhexamethylene biguanide according to claim 14, wherein said buffer system is selected from the group consisting of disodium phosphate dodecahydrate/monobasic sodium phosphate, disodium phosphate dodecahydrate/citric acid, Tris/HCl or boric acid/borate.

16. The process for the preparation of a liquid formulation based on polyhexamethylene biguanide according to any of claims 13-15, wherein the isotonizing agent is sodium chloride or potassium chloride.

17. An eye lens loaded with the liquid formulation based on polyhexamethylene biguanide according to any of claims 1-11, for the controlled release of the active ingredient for use in the treatment of *Acanthamoeba* keratitis, co-infections by *Acanthamoeba* and *Pseudomonas aeruginosa* or fungal infections.

## Patentansprüche

1. Flüssige Formulierung auf Basis von Polyhexamethylenbiguanid, umfassend Polyhexamethylenbiguanid in einer Konzentration im Bereich von 0,04%-0,08% (w/v), ein Puffersystem zum Halten des **pH** im Bereich von 5-6,5, ein Isotonisierungsmittel, um die Osmolarität im Bereich von 270-330 mOsm/Kg zu halten, **gekennzeichnet durch** ein Molekulargewicht von Polyhexamethylenbiguanid im Bereich von 2.300-6.000 amu und einen Polydispersionsindex des Polymers im Bereich von 1.5-1,9, geeignet zur ophthalmischen Verabreichung zur Verwendung bei der Behandlung von Acanthamoeba-Keratitis und/oder Pilzinfektionen.

2. Flüssige Formulierung auf Basis von Polyhexamethylenbiguanid zur Verwendung nach Anspruch 1 zur Behandlung von Co-Infektionen durch *Acanthamoeba* und *Pseudomonas aeruginos* oder *Staphylococcus epidermis.*

3. Flüssige Formulierung auf Basis von Polyhexamethylenbiguanid zur Verwendung nach Anspruch 1 bis 2, wobei das Puffersystem aus der Gruppe ausgewählt ist, die Phosphat, Citrat, Bicarbonat, Borat, Tris, Glycerin, Mannit, Sorbit, Trometamol, Histidin, Glycin, HEPES-Puffer oder ein gemischtes System umfasst.

4. Flüssige Formulierung auf Basis von Polyhexamethylenbiguanid zur Verwendung nach Anspruch 3, wobei das Puffersystem aus der Gruppe ausgewählt ist, die aus Dinatriumphosphat-Dodecahydrat/einbasigem Natriumphosphat, Dinatriumphosphat-Dodecahydrat/Zitronensäure, Tris/HCl oder Borsäure/Borat besteht.

5. Flüssige Formulierung auf Basis von Polyhexamethylenbiguanid zur Verwendung nach einem der Ansprüche 1-4, wobei das Isotonisierungsmittel Natriumchlorid oder Kaliumchlorid ist.

6. Flüssige Formulierung auf Basis von Polyhexamethylenbiguanid zur Verwendung nach einem der Ansprüche 1-5, **gekennzeichnet durch** einen Polydispersitätsindex des Polymers im Bereich von 1,7-1,8.

7. Flüssige Formulierung auf Basis von Polyhexamethylenbiguanid zur Verwendung nach einem der Ansprüche 1-6 mit einem pH-Wert von 5,8.

8. Flüssige Formulierung auf Basis von Polyhexamethylenbiguanid zur Verwendung nach einem der Ansprüche 1-7, in Form eines Collyriums, von Augentropfen, eines ophthalmischen Gels oder von viskosen Lösungen.

9. Flüssige Formulierung auf Basis von Polyhexamethylenbiguanid zur Verwendung nach Anspruch 8 in Form eines ophthalmischen Gels oder einer Viskoselösung, ferner umfassend einen Viskositätsmittel, das aus der Gruppe ausgewählt ist, die aus Xanthangummi, Gellangummi, Polyvinylalkohol, Hyaluronsäure, Natriumhyaluronat, Carboxymethylcellulose und Hydroxypropylcellulose besteht.

10. Flüssige Formulierung auf Basis von Polyhexamethylenbiguanid zur Verwendung nach einem der Ansprüche 1-9, ferner umfassend einen Penetrationsverstärker, ausgewählt aus Tween 80 und Benzalkoniumchlorid.

11. Flüssige Formulierung auf Basis von Polyhexamethylenbiguanid zur Verwendung nach einem der Ansprüche 1-10, ferner umfassend 0,02 % Chlorhexidin, 0,1 % Propamidin oder 0,1 % Desomidin.

12. Flüssige Formulierung auf Basis von Polyhexamethylenbiguanid zur Verwendung nach einem der Ansprüche 1-11 durch ophthalmische Verabreichung einer graduierten Dosierung von 16 Tropfen/Tag für 5 Tage, 8 Tropfen/Tag für 7 Tage, 6 Tropfen/Tag für 7 Tage und 4 Tropfen/Tag bis zur klinischen Abheilung.

13. Verfahren zur Herstellung einer flüssigen Formulierung auf Basis von Polyhexamethylenbiguanid nach einem der Ansprüche 1-12, umfassend die folgenden Schritte:
(i) Zugabe des Puffersystems in einer Konzentration von 0,01 % bis 4,5 % (w/v) und des Isotonisierungsmittels in einer Konzentration von 0,01 % bis 5,5 % (w/v) in gereinigtem Wasser unter sanftem und kontinuierlichem Rühren; gegebenenfalls Zugabe eines Viskositätsmittels, ausgewählt aus Natriumhyaluronat, Xanthangummi, Polyvinylalkohol, Carboxymethylcellulose und Hydroxypropylcellulose;
(ii) Zugabe von Polyhexamethylenbiguanid mit einem Molekulargewicht im Bereich von 2.300-6.000 amu und einem Polydispersionsindex im Bereich von 1,5-1,9, vorzugsweise 1,7-1,8, in einer Konzentration im Bereich von 0,04%-0,08% (w/v) unter leichtem Rühren;
(iii) durch Zugabe von gereinigtem Wasser auf Volumen bringen;
(iv) Sterilisation durch Filtration oder Hitze.

14. Verfahren zur Herstellung einer flüssigen Formulierung auf Basis von Polyhexamethylenbiguanid nach Anspruch 13, wobei das Puffersystem aus der Gruppe ausgewählt ist, die Phosphat, Citrat, Bicarbonat, Borat, Tris, Glycerin, Mannit, Sorbit, Trometamol, Histidin, Glycin, HEPES-Puffer oder ein gemischtes System umfasst.

15. Verfahren zur Herstellung einer flüssigen Formulierung auf Basis von Polyhexamethylenbiguanid nach Anspruch 14, wobei das Puffersystem aus der Gruppe ausgewählt ist, die aus Dinatriumphosphat-Dodecahydrat/einbasigem Natriumphosphat, Dinatriumphosphat-Dodecahydrat/Zitronensäure, Tris/HCl oder Borsäure/Borat besteht.

16. Verfahren zur Herstellung einer flüssigen Formulierung auf Basis von Polyhexamethylenbiguanid nach einem der Ansprüche 13-15, **dadurch gekennzeichnet, dass** das Isotonisierungsmittel Natriumchlorid oder Kaliumchlorid ist.

17. Augenlinse, beladen mit der flüssigen Formulierung auf Basis von Polyhexamethylenbiguanid nach einem der Ansprüche 1-11 zur kontrollierten Freisetzung des Wirkstoffs zur Verwendung bei der Behandlung von *Acanthamoeba-Keratitis,* Co-Infektionen durch *Acanthamoeba* und *Pseudomonas aeruginosa* oder Pilzinfektionen.

## Revendications

1. Formulation liquide à base de polyhexaméthylène biguanide comprenant du polyhexaméthylène biguanide à une concentration dans la plage de 0,04% à 0,08% (p/v), un système tampon pour maintenir le **pH** dans la plage de 5 à 6,5, un agent isotonisant pour maintenir l'osmolarité dans la plage de 270 à 330 mOsm/Kg, **caractérisée par** un poids moléculaire du polyhexaméthylène biguanide dans la plage de 2 300 à 6 000 amu et un indice de polydispersion du polymère dans la plage de 1,5 à 1,9, convenant à l'administration ophtalmique pour le traitement de *Acanthamoeba* kératites et/ou d'infections fongiques.

2. Formulation liquide à base de polyhexaméthylène biguanide pour utilisation selon la revendication 1, pour le traitement des co-infections par *Acanthamoeba* et *Pseudomonas aeruginos* ou *Staphylococcus epidermis.*

3. Formulation liquide à base de polyhexaméthylène biguanide pour utilisation selon les revendications 1 à 2, dans laquelle le système tampon est choisi dans le groupe comprenant le phosphate, le citrate, le bicarbonate, le borate, le Tris, le glycérol, le mannitol, le sorbitol, le trométamol, l'histidine, la glycine, le tampon HEPES, ou un système mixte.

4. Formulation liquide à base de polyhexaméthylène biguanide pour utilisation selon la revendication 3, dans laquelle ledit système tampon est choisi dans le groupe consistant en phosphate disodique dodécahydraté/phosphate monobasique de sodium, phosphate disodique dodécahydraté/acide citrique, Tris/HCl ou acide borique/borate.

5. Formulation liquide à base de polyhexaméthylène biguanide pour utilisation selon l'une des revendications 1 à 4, dans laquelle l'agent isotonisant est le chlorure de sodium ou le chlorure de potassium.

6. Formulation liquide à base de polyhexaméthylène biguanide pour utilisation selon l'une des revendications 1 à 5, **caractérisée par** un indice de polydispersité du polymère dans la plage de 1,7 à 1,8.

7. Formulation liquide à base de polyhexaméthylène biguanide utilisée selon l'une des revendications 1 à 6, ayant un pH de 5,8.

8. Formulation liquide à base de polyhexaméthylène biguanide pour utilisation selon l'une des revendications 1 à 7, sous forme de collyre, de gouttes pour les yeux, de gel ophtalmique ou de solutions visqueuses.

9. Formulation liquide à base de polyhexaméthylène biguanide pour utilisation selon la revendication 8, sous forme de gel ophtalmique ou de solution visqueuse, comprenant en outre un agent viscosifiant choisi dans le groupe constitué par la gomme xanthane, la gomme gellane, l'alcool polyvinylique, l'acide hyaluronique, le hyaluronate de sodium, la carboxyméthylcellulose et l'hydroxypropylcellulose.

10. Formulation liquide à base de polyhexaméthylène biguanide pour utilisation selon l'une des revendications 1 à 9, comprenant en outre un activateur de pénétration choisi parmi le Tween 80 et le chlorure de benzalkonium.

11. Formulation liquide à base de polyhexaméthylène biguanide utilisée selon l'une des revendications 1 à 10, comprenant en outre 0,02 % de chlorhexidine, 0,1 % de propamidine ou 0,1 % de désomidine.

12. Formulation liquide à base de polyhexaméthylène biguanide utilisée selon l'une des revendications 1 à 11, par administration ophtalmique d'une dose graduée de 16 gouttes/jour pendant 5 jours, 8 gouttes/jour pendant 7 jours, 6 gouttes/jour pendant 7 jours et 4 gouttes/jour jusqu'à résolution clinique.

13. Procédé de préparation d'une formulation liquide à base de polyhexaméthylène biguanide selon l'une quelconque des revendications 1 à 12, comprenant les étapes suivantes :
(i) ajout du système tampon à une concentration comprise entre 0,01 % et 4,5 % (p/v) et de l'agent isotonisant à une concentration comprise entre 0,01 % et 5,5 % (p/v) dans de l'eau purifiée sous agitation douce et continue ; ajout facultatif d'un agent viscosifiant choisi parmi le hyaluronate de sodium, la gomme xanthane, l'alcool polyvinylique, la carboxyméthylcellulose et l'hydroxypropylcellulose ;
(ii) ajout de polyhexaméthylène biguanide ayant un poids moléculaire dans la plage de 2 300 à 6 000 amu et un indice de polydispersion dans la plage de 1,5 à 1,9, de préférence de 1,7 à 1,8, à une concentration dans la plage de 0,04 % à 0,08 % (p/v) sous une légère agitation ;
(iii) apport de volume par ajout d'eau purifiée ;
(iv) stérilisation par filtration ou thermique.

14. Procédé de préparation d'une formulation liquide à base de polyhexaméthylène biguanide selon la revendication 13, dans lequel ledit système tampon est choisi dans le groupe comprenant le phosphate, le citrate, le bicarbonate, le borate, le Tris, le glycérol, le mannitol, le sorbitol, le trométamol, l'histidine, la glycine, le tampon HEPES, ou un système mixte.

15. Procédé de préparation d'une formulation liquide à base de polyhexaméthylène biguanide selon la revendication 14, dans lequel ledit système tampon est choisi dans le groupe constitué par le phosphate disodique dodécahydraté/phosphate monobasique de sodium, le phosphate disodique dodécahydraté/acide citrique, le Tris/HCl ou l'acide borique/borate.

16. Procédé de préparation d'une formulation liquide à base de polyhexaméthylène biguanide selon l'une quelconque des revendications 13 à 15, dans lequel l'agent isotonisant est le chlorure de sodium ou le chlorure de potassium.

17. Lentille oculaire chargée de la formulation liquide à base de polyhexaméthylène biguanide selon l'une des revendications 1 à 11, pour la libération contrôlée de l'ingrédient actif en vue d'une utilisation dans le traitement de *Acanthamoeba* kératite, co-infections par *Acanthamoeba* et *Pseudomonas aeruginosa* ou d'infections fongiques.
